# EUROPEAN PATENT APPLICATION

(11) **EP 4 790 070 A1**
(43) Date of publication of application: **12.08.2026**
(21) Application number: 24874996.2
(22) Date of filing: 04.10.2024
(51) Int. Cl.: C07K 16/28, A61P 35/00, A61K 39/00

(54) **ANTI-CD24 ANTIBODY OR ANTIGEN-BINDING FRAGMENT THEREOF, AND USE THEREOF**

(30) Priority: 04.10.2023 KR 20230131985
(71) Applicant: Daegu Gyeongbuk Institute of Science and Technology, Daegu 42988 (KR)
(72) Inventor: YEA, Kyung Moo, Daegu 43016 (KR); SHIN, Sang Hee, Daegu 42995 (KR); SHIN, Ji Won, Busan 46532 (KR)
(74) Representative: Caspary, Karsten
(86) International application number: PCT/KR2024/015109
(87) International publication number: WO 2025/075440

(57) **Abstract**

The present disclosure relates to a novel anti-CD24 antibody and a use thereof, and a CD24-specific antibody according to one aspect increases the phagocytic activity of intratumoral macrophages, thereby enabling its utilization as a broad-spectrum anticancer agent for effectively preventing or treating various CD24-related cancers regardless of the specific cancer type, and has high binding affinity for both human and mouse CD24, enabling its application in both preclinical and clinical settings.

## Description

### Technical Field

The present disclosure relates to a novel anti-CD24 antibody and a use thereof.

### Background Art

Among "don't eat me" signals, the most extensively studied antiphagocytic protein is CD47, and therapeutic antibodies targeting CD47 have shown promising anti-tumor efficacy in preclinical and clinical settings; however, they are associated with off-target toxicities such as anemia and thrombocytopenia.

CD24, which is a recently discovered "don't eat me" signal, has been reported to enable immune evasion and protect tumor cells from immune attack through interaction with Siglec-10 on immune cells. CD24 is overexpressed in 68 % of human cancers and exhibits higher expression compared to other immune checkpoints, while its expression level is low in normal tissues and human erythrocytes. Therefore, a monoclonal antibody targeting CD24 can induce anti-tumor effects in various types of cancer by blocking the interaction with Siglec-10 to increase phagocytosis, while avoiding the side effects associated with CD47.

CD24 was first identified as an immune checkpoint in 2019; prior to that, antibodies targeting CD24 were developed for tumor targeting purposes. That is, previously reported antibodies were limited to targeting CD24 overexpressed on cancer cells, and it was unclear whether they were capable of blocking the interaction with Siglec-10 on immune cells. Furthermore, most of these antibodies are murine monoclonal IgG antibodies and thus have the disadvantage of being unsuitable for clinical application.

The present inventors have made research efforts to provide a novel anti-CD24 antibody and have developed a novel antibody that can provide a potent anti-tumor effect regardless of the cancer type by increasing the phagocytic activity of macrophages. This antibody is applicable to both humans and mice, allowing for application in both preclinical and clinical settings.

### Disclosure of Invention

### Technical Problem

One aspect of the present disclosure provides an antibody or antigen-binding fragment thereof that specifically binds to CD24.

Another aspect of the present disclosure provides a gene encoding the antibody or antigen-binding fragment thereof; a vector including the gene; or a recombinant cell including the gene or the vector.

Another aspect of the present disclosure provides an antibody-drug conjugate (ADC) including the antibody or antigen-binding fragment thereof.

Another aspect of the present disclosure provides a pharmaceutical composition for preventing or treating cancer, including the antibody or antigen-binding fragment thereof, or the antibody-drug conjugate.

Another aspect of the present disclosure provides a method of preventing or treating cancer, the method including administering an effective amount of the antibody or antigen-binding fragment thereof that specifically binds to CD24, or the antibody-drug conjugate, to a subject in need thereof.

Another aspect of the present disclosure provides use of the antibody or antigen-binding fragment thereof, or the antibody-drug conjugate, in the manufacture of a pharmaceutical preparation for preventing or treating cancer.

### Solution to Problem

One aspect provides an antibody or antigen-binding fragment thereof that specifically binds to CD24.

As used herein, the terms "antibody," "antigen-binding region or site," and "antigen-binding polypeptide" refer to a polypeptide or polypeptide complex that specifically recognizes and binds to an antigen. Since the present disclosure relates to a CD24 antibody that specifically binds to CD24, the term "antibody" as used herein without modification may refer to an antibody that specifically binds to CD24, unless otherwise specified.

The antibody or antigen-binding fragment thereof of the present disclosure may be a whole antibody, or any antigen-binding fragment or single chain thereof. The term "whole antibody" refers to a structure in which two full-length light chains and two full-length heavy chains are connected by disulfide bonds. The whole antibody includes IgA, IgD, IgE, IgM, and IgG, and the IgG includes IgG1, IgG2, IgG3, and IgG4 as subtypes thereof.

As used herein, the term "antigen-binding fragment" refers to a fragment of the whole structure of an antibody and is a portion of an antibody containing a part capable of binding to an antigen.

The antibody or antigen-binding fragment thereof of the present disclosure may also be any protein- or peptide-containing molecule containing at least a portion of an immunoglobulin molecule having a biological activity of binding to an antigen out of the whole antibody. Such examples include, but are not limited to, a complementarity determining region (CDR) of a heavy chain or a light chain or a ligand-binding portion thereof, a heavy chain or light chain variable region, a heavy chain or light chain constant region, a framework region (FR), or any portion thereof, or at least one portion of a binding protein.

As used herein, the term "heavy chain (HC or CH)" refers to a full-length heavy chain and a fragment thereof including a variable region domain VH containing an amino acid sequence having a variable region (VR) sequence sufficient to confer specificity to an antigen, and three constant region domains CH1, CH2, and CH3.

As used herein, the term "light chain (LC or CL)" refers to a full-length light chain and a fragment thereof including a variable region domain VL containing an amino acid sequence having a variable region sequence sufficient to confer specificity to an antigen, and a constant region domain CL.

The term "complementarity determining region (CDR; i.e., CDR1, CDR2, and CDR3)" as used herein refers to a region within the variable region of an antibody that has binding specificity for an antigen. Each variable region typically has three CDR regions identified as CDR1, CDR2, and CDR3. As used herein, a CDR included in a heavy chain variable region is denoted as "H-CDR," and a CDR included in a light chain variable region is denoted as "L-CDR."

In an embodiment, the antibody or antigen-binding fragment thereof may include one or more selected from the group consisting of a monoclonal antibody, a domain antibody (dAb), a single chain antibody (scAb), a Fab fragment, a Fab' fragment, an F(ab')2 fragment, a scFab fragment, an Fv fragment, a dsFv fragment, a single-chain variable fragment (scFv), a scFv-Fc fragment, a single domain heavy chain antibody, a single domain light chain antibody, an antibody variant, a multimeric antibody, a minibody, a diabody, a triabody, a tetrabody, a Bis-scFv, a nanobody, a bispecific antibody, and a multispecific antibody. Preferably, the antibody of the present disclosure may be an antibody including an scFv or an scFv-Fc fragment.

As used herein, the term "Fc" region may include two heavy chain fragments containing the CH2 and CH3 domains of an antibody. These two heavy chain fragments may be combined with each other by two or more disulfide bonds and hydrophobic interactions of the CH3 domain.

As used herein, the term "Fab fragment" may consist of one heavy chain and one light chain containing only the CH1 and variable regions. The heavy chain of the Fab molecule may not form a disulfide bond with another heavy chain molecule.

As used herein, the term 'Fab' fragment' includes a region between the CH1 and CH2 domains of the heavy chain in addition to the Fab fragment, which may form an F(ab')₂ molecule by forming a disulfide bond between the two heavy chains of two Fab' fragment molecules.

As used herein, the term "F(ab')₂ fragment" includes two heavy chains and two light chains containing the variable regions, CH1, and a portion of the constant region between the CH1 and CH2 domains as described above, whereby an intrachain disulfide bond is formed between the two heavy chains. Accordingly, the F(ab')₂ fragment consists of two Fab' fragments, and the two Fab' fragments may be bonded to each other by the disulfide bond therebetween.

The term "Fv fragment" as used herein may be an antibody including the respective variable regions of the heavy chain and the light chain, but not including the constant regions. The terms "single-chain variable fragment," "single-chain Fv," or "scFv" antibody fragment may refer to a fusion protein including the VH and VL domains of an antibody, where these domains exist within a single polypeptide chain. The Fv polypeptide may further include a polypeptide linker between the VH domain and the VL domain that allows the scFv to form a desired structure for antigen binding. The scFv-Fc may be one in which an Fc is linked to an scFv. A diabody may include two molecules of an scFv.

In an embodiment, an antibody specifically binding to CD24 of the present disclosure includes a heavy chain complementarity determining region (H-CDR) including H-CDR1, H-CDR2, and H-CDR3; and a light chain complementarity determining region (L-CDR) including L-CDR1, L-CDR2, and L-CDR3, and may include:
an H-CDR1 including an amino acid sequence of SEQ ID NO: 1 or 2; a portion thereof; or an amino acid sequence having at least 75 %, 80 %, 85 %, 90 %, 95 %, 96 %, 97 %, 98 %, or 99 % homology or identity therewith;
an H-CDR2 including an amino acid sequence of SEQ ID NO: 3 or 4; a portion thereof; or an amino acid sequence having at least 75 %, 80 %, 85 %, 90 %, 95 %, 96 %, 97 %, 98 %, or 99 % homology or identity therewith;
an H-CDR3 including an amino acid sequence of SEQ ID NO: 5 or 6; a portion thereof; or an amino acid sequence having at least 75 %, 80 %, 85 %, 90 %, 95 %, 96 %, 97 %, 98 %, or 99 % homology or identity therewith;
an L-CDR1 including an amino acid sequence of SEQ ID NO: 7 or 8; a portion thereof; or an amino acid sequence having at least 75 %, 80 %, 85 %, 90 %, 95 %, 96 %, 97 %, 98 %, or 99 % homology or identity therewith;
an L-CDR2 including an amino acid sequence of sequence AAS or sequence EVT; a portion thereof; or an amino acid sequence having at least 75 %, 80 %, 85 %, 90 %, 95 %, 96 %, 97 %, 98 %, or 99 % homology or identity therewith; and
an L-CDR3 including an amino acid sequence of SEQ ID NO: 9 or 10; a portion thereof; or an amino acid sequence having at least 75 %, 80 %, 85 %, 90 %, 95 %, 96 %, 97 %, 98 %, or 99 % homology or identity therewith.

In an embodiment, the heavy chain variable region may include an H-CDR1 including an amino acid sequence of SEQ ID NO: 1; an H-CDR2 including an amino acid sequence of SEQ ID NO: 3; and an H-CDR3 including an amino acid sequence of SEQ ID NO: 5.

In an embodiment, the heavy chain variable region may include an H-CDR1 including an amino acid sequence of SEQ ID NO: 2; an H-CDR2 including an amino acid sequence of SEQ ID NO: 4; and an H-CDR3 including an amino acid sequence of SEQ ID NO: 6.

In an embodiment, the light chain variable region may include an L-CDR1 including an amino acid sequence of SEQ ID NO: 7; an L-CDR2 including the amino acid sequence of sequence AAS; and an L-CDR3 including an amino acid sequence of SEQ ID NO: 9.

In an embodiment, the light chain variable region may include an L-CDR1 including an amino acid sequence of SEQ ID NO: 8; an L-CDR2 including the amino acid sequence of sequence EVT; and an L-CDR3 including an amino acid sequence of SEQ ID NO: 10.

In an embodiment, the antibody or antigen-binding fragment thereof may include an H-CDR1 including an amino acid sequence of SEQ ID NO: 1; an H-CDR2 including an amino acid sequence of SEQ ID NO: 3; an H-CDR3 including an amino acid sequence of SEQ ID NO: 5; an L-CDR1 including an amino acid sequence of SEQ ID NO: 7; an L-CDR2 including the amino acid sequence AAS; and an L-CDR3 including an amino acid sequence of SEQ ID NO: 9.

In an embodiment, the antibody or antigen-binding fragment thereof may include an H-CDR1 including an amino acid sequence of SEQ ID NO: 2; an H-CDR2 including an amino acid sequence of SEQ ID NO: 4; an H-CDR3 including an amino acid sequence of SEQ ID NO: 6; an L-CDR1 including an amino acid sequence of SEQ ID NO: 8; an L-CDR2 including the amino acid sequence EVT; and an L-CDR3 including an amino acid sequence of SEQ ID NO: 10.

In an embodiment, the antibody or antigen-binding fragment thereof may include a heavy chain variable region including an amino acid sequence of SEQ ID NO: 11 or 12; a portion thereof; or an amino acid sequence having at least 75 %, 80 %, 85 %, 90 %, 95 %, 96 %, 97 %, 98 %, or 99 % homology or identity therewith; and
a light chain variable region including an amino acid sequence of SEQ ID NO: 13 or 14; a portion thereof; or an amino acid sequence having at least 75 %, 80 %, 85 %, 90 %, 95 %, 96 %, 97 %, 98 %, or 99 % homology or identity therewith.

In an embodiment, the antibody or antigen-binding fragment thereof may include a heavy chain variable region including an amino acid sequence of SEQ ID NO: 11; and a light chain variable region including an amino acid sequence of SEQ ID NO: 13.

In an embodiment, the antibody or antigen-binding fragment thereof may include a heavy chain variable region including an amino acid sequence of SEQ ID NO: 12; and a light chain variable region including an amino acid sequence of SEQ ID NO: 14.

In an embodiment, the antibody or antigen-binding fragment thereof may include any one or more amino acid sequences selected from the group consisting of SEQ ID NOs: 15 to 18; a portion thereof; or an amino acid sequence having at least 75 %, 80 %, 85 %, 90 %, 95 %, 96 %, 97 %, 98 %, or 99 % homology or identity therewith.

In an embodiment, the heavy chain variable region and the light chain variable region may be linked directly or via a linker.

The antibody or antigen-binding fragment thereof of the present disclosure, which specifically binds to CD24, may include "variants." The variants may refer to a polypeptide in which one or more amino acid residues are inserted, deleted, added, and/or substituted from the polypeptide sequence. Accordingly, as long as the desired biological activity and/or structure of the antibody and/or antigen-binding fragment thereof are maintained, the antibody or fragment may include a fusion polypeptide by being linked to another polypeptide. The variants may have a sequence identity of 80 % or more, 85 % or more, 90 % or more, 95 % or more, 96 % or more, 97 % or more, 98 % or more, or 99 % or more relative to the sequence of the antibody or antigen-binding fragment thereof; and may have, for example, a sequence identity of about 99 %, 98 %, 97 %, 96 %, 95 %, 94 %, 93 %, 92 %, 91 %, 90 %, 89 %, 88 %, 87 %, 86 %, 85 %, 84 %, 83 %, 82 %, 81 %, or 80 %.

The terms "homology" or "identity" as used herein may refer to an overall relatedness between nucleic acid molecules and/or between polypeptides. In an embodiment, nucleic acids or polypeptides may be considered to be "substantially identical" to each other if their sequences are at least 60 %, 65 %, 70 %, 75 %, 80 %, 85 %, 90 %, 95 %, 96 %, 97 %, 98 %, or 99 % identical. The calculation of percent identity between two nucleic acid or polypeptide sequences may be performed, for example, by aligning the two sequences for optimal comparison purposes. In a specific embodiment, the length of the sequences aligned for comparison purposes may be at least 30 %, at least 40 %, at least 50 %, at least 60 %, at least 70 %, at least 75 %, at least 80 %, at least 85 %, at least 90 %, at least 95 %, at least 96 %, at least 97 %, at least 98 %, at least 99 %, or substantially 100 % of the length of a reference sequence.

The antibody or antigen-binding fragment thereof of the present disclosure does not occur naturally and may be synthesized chemically or recombinantly. Specifically, the antibody or fragment may be synthesized by linking an Fc fragment to an scFv fragment via a linker.

In an embodiment, the antibody or antigen-binding fragment thereof may be a mouse antibody, a chimeric antibody, a humanized antibody, or a fully human antibody.

The antibody or antigen-binding fragment thereof of the present disclosure may have high binding affinity for both human CD24 and mouse CD24. That is, the antibody of the present disclosure is applicable to both clinical and preclinical applications.

The antibody or antigen-binding fragment thereof of the present disclosure may include not only the sequence of the anti-CD24 antibody but also biological equivalents thereof, and may be modified, within a range capable of specifically recognizing CD24. For example, additional changes may be made to the amino acid sequence of the antibody to further improve the binding affinity and/or other biological properties of the antibody. Such modifications include, for example, deletion, insertion, and/or substitution of amino acid sequence residues of the antibody. Such amino acid mutations are made based on the relative similarity of amino acid side-chain substituents, such as hydrophobicity, hydrophilicity, charge, size, and the like. In addition, the antibody or antigen-binding fragment thereof may be modified by conjugation or binding, glycosylation, tag attachment, or a combination thereof.

Another aspect provides a gene encoding the antibody or antigen-binding fragment thereof that specifically binds to CD24.

Another aspect provides a vector including the gene.

As used herein, the term "encoding" refers to the inherent property of a specific nucleotide sequence in a polynucleotide, such as a gene, a cDNA, or an mRNA, serving as a template for the synthesis of other polymers and macromolecules in biological processes having either a defined sequence of nucleotides (i.e., rRNA, tRNA, and mRNA) or a defined sequence of amino acids, and the biological properties resulting therefrom. Thus, a gene encodes a protein if the transcription and translation of mRNA corresponding to the gene produces the protein in a cell or other biological system. Both the coding strand, which is the nucleotide sequence identical to the mRNA sequence and usually provided in the sequence listing, and the non-coding strand used as a template for transcription of the gene or cDNA, can be referred to as encoding the protein or other product of the gene or cDNA.

As used herein, the term "vector" refers to any nucleic acid construct capable of delivering foreign genetic material or directing the migration thereof to a target cell in which the polynucleotide can be replicated and/or expressed. The vector includes a construct to be delivered. The vector may be a linear molecule or a circular molecule. The vector may be an integrating or non-integrating vector.

In an embodiment, the vector may be any one selected from the group consisting of a plasmid, a cosmid, a virus, a phage, a recombinant expression cassette, and a transposon. However, the vector is not limited thereto, and it will be understood that any suitable vector known in the art may be used if it is consistent with the purpose of the present disclosure.

It will be understood that a gene sequence in which a portion of the sequence is deleted, modified, substituted, or added from the gene or the vector of the present disclosure may also be used in the present application, provided that it has a function identical or corresponding to that of the gene of the present disclosure.

In an embodiment, the gene may include a gene sequence of SEQ ID NO: 20 or 21, a portion thereof, or a nucleotide sequence having at least 70 %, 75 %, 80 %, 85 %, 90 %, 95 %, 96 %, 97 %, 98 %, or 99 % identity therewith.

Another aspect provides a recombinant cell including the gene or the vector.

The recombinant cell may include a single vector containing a single gene encoding the antibody or antigen-binding fragment thereof. The recombinant cell may be one into which a single vector including genes encoding the light chain and the heavy chain of the antibody or antigen-binding fragment thereof, respectively, has been introduced; or one into which a plurality of vectors, each including the respective gene, have been introduced. Each gene or vector may independently be introduced into the recombinant cell simultaneously, sequentially, or in reverse order.

In an embodiment, the recombinant cell may be an archaeal cell, a bacterial cell, a eukaryotic cell, a eukaryotic unicellular organism, a somatic cell, a germ cell, a stem cell, a plant cell, an animal cell, a mammalian cell, a mouse cell, a non-human primate cell, or a human cell.

Another aspect provides an antibody-drug conjugate (ADC) including the antibody or antigen-binding fragment thereof that specifically binds to CD24.

As used herein, "antibody-drug conjugate (ADC)" may refer to a substance having a structure in which a cytotoxic small molecule drug (payload) is conjugated to an antibody or an antigen-binding fragment thereof that binds to a specific target antigen on a cell surface.

In an embodiment, the antibody-drug conjugate may be formed by conjugating a therapeutic agent (drug) to the antibody or antigen-binding fragment thereof having target specificity via a linker. That is, the antibody-drug conjugate may include the antibody or antigen-binding fragment thereof specifically binding to CD24; a drug moiety; and a linker linking the antibody or antigen-binding fragment thereof and the drug moiety.

The linker may include a cleavable linker or a non-cleavable linker. The cleavable linker, such as a peptide linker, may be cleaved by an intracellular peptidase or protease enzyme, for example, a lysosomal or endosomal protease. For example, the cleavable linker may be any one selected from the group consisting of a protease-cleavable linker, an acid-cleavable linker, a disulfide linker, a β-glucuronide-based linker, and a β-galactoside-based linker, but is not limited thereto. With regard to the non-cleavable linker, the drug may be released after the antibody is non-selectively degraded by intracellular hydrolysis.

In an embodiment, the drug or therapeutic agent may be any one selected from the group consisting of a chemotherapeutic compound, a cytotoxic compound, an immunomodulatory compound, an anticancer agent, an antiviral agent, an antibacterial agent, an antifungal agent, an antiparasitic agent, and a combination thereof. For example, the cytotoxic compound may be any one selected from the group consisting of a tubulin inhibitor (mitotic inhibitor), a DNA alkylating agent, a topoisomerase inhibitor, and a combination thereof, but is not limited thereto. More specifically, the therapeutic agent may be MMAE_Protein A, cotinine-duocarmycin, or Herceptin, but is not limited thereto.

Another aspect provides a pharmaceutical composition for the prevention or treatment of cancer, including the antibody or antigen-binding fragment thereof that specifically binds to CD24; or the antibody-drug conjugate.

Another aspect provides a method for preventing or treating cancer, including the step of administering an effective amount of the antibody or antigen-binding fragment thereof that specifically binds to CD24; or the antibody-drug conjugate to a subject in need thereof.

Another aspect provides a use of the antibody or antigen-binding fragment thereof; or the antibody-drug conjugate for the manufacture of a pharmaceutical agent for the prevention or treatment of cancer.

As used herein, "prevention" refers to all actions that inhibit or delay a disease by administering the composition of the present disclosure to a subject. For preventive benefit, the composition may be administered to a subject at risk of developing a specific disease, disorder, or symptom, or to a subject reporting one or more physiological symptoms of a disease, even if the disease, disorder, or symptom has not yet appeared.

As used herein, "treatment" refers to all actions that improve or beneficially affect the symptoms of a disease by administering the composition of the present disclosure to a subject. As used herein, "treatment," "alleviation," or "improvement" may be used interchangeably. A therapeutic benefit refers to any therapeutically significant improvement in or effect on one or more diseases, disorders, or symptoms under treatment.

The antibody or antigen-binding fragment thereof, or the antibody-drug conjugate, may increase the phagocytic activity of intratumoral macrophages. The antibody of the present disclosure may enhance the phagocytic capacity of macrophages toward cancer cells and their infiltration capability into tumor tissues. In addition, the antibody of the present disclosure may increase the expression of TNFα, which is a representative activity marker of anti-tumor M1 macrophages, and Granzyme B and IFNγ, which are representative activity markers of cytotoxic T cells (CD8+ T cells).

As used herein, "cancer" or tumor may collectively refer to a disease caused by cells having aggressive characteristics of dividing and proliferating while ignoring normal growth limits, invasive characteristics of infiltrating surrounding tissues, and metastatic characteristics of spreading to other parts of the body. The cancer may be a solid cancer or a hematological cancer.

For example, the cancer may be pseudomyxoma peritonei, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell tumor, male breast cancer, brain cancer, pituitary adenoma, multiple myeloma, gallbladder cancer, biliary tract cancer, colorectal cancer, chronic myeloid leukemia, chronic lymphocytic leukemia, retinoblastoma, choroidal melanoma, cancer of the Ampulla of Vater, bladder cancer, peritoneal cancer, parathyroid cancer, adrenal cancer, nasal cavity and paranasal sinus cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureter cancer, urethral cancer, cancer of unknown primary origin, gastric lymphoma, stomach cancer, gastric carcinoid tumor, gastrointestinal stromal tumor, Wilms' tumor, breast cancer, triple-negative breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid tumor, vaginal cancer, spinal cord cancer, acoustic neuroma, pancreatic cancer, salivary gland cancer, Kaposi's sarcoma, Paget's disease, tonsil cancer, squamous cell carcinoma, lung adenocarcinoma, lung cancer, squamous cell lung carcinoma, skin cancer, anal cancer, rhabdomyosarcoma, laryngeal cancer, pleural cancer, hematological cancer, or thymic cancer.

Specifically, the cancer may include one or more selected from the group consisting of breast cancer, lung cancer, skin cancer, kidney cancer, colorectal cancer, head and neck cancer, stomach cancer, colon cancer, prostate cancer, bladder cancer, rectal cancer, thyroid cancer, liver cancer, cervical cancer, melanoma, rectal cancer, anal cancer, urethral cancer, ovarian cancer, esophageal cancer, and pancreatic cancer.

The pharmaceutical composition of the present disclosure may be in any form suitable for the intended method of administration. As used herein, "administration" refers to introducing a predetermined substance into a patient by any appropriate method.

The pharmaceutical composition of the present disclosure may be administered in a pharmaceutically effective amount. A "pharmaceutically effective amount" refers to an amount sufficient to treat or prevent a disease at a reasonable benefit/risk ratio applicable to medical treatment or prevention, and the amount may be adjusted according to factors, such as the type and severity of the patient's disease, the type of active ingredient and formulation being administered, the patient's age, gender, weight, health status, diet, and sensitivity, the time and method of administration, the combination of the composition or concurrently used drugs, and other factors well known in the medical field.

The dosage of the pharmaceutical composition for the prevention or treatment of a disease according to the present disclosure may be in the range of 0.01 ug/kg to 10 g/kg per day, specifically in the range of 0.01 mg/kg to 1 g/kg, depending on the patient's condition, weight, gender, age, severity, and route of administration. The administration may be performed once a day or divided into several administrations per day. These dosages should not be construed as limiting the scope of the present disclosure in any aspect.

The dosage may be variously prescribed based on factors such as formulation method, administration mode, age, weight, gender, pathological condition, diet, administration time, administration route, excretion rate, and response sensitivity of the patient; and those skilled in the art can appropriately adjust the dosage in consideration of these factors. The frequency of administration may be once a day, or two or more times within the range of clinically acceptable side effects. Regarding the site of administration, the composition may be administered at one or more locations. The total number of administration days per treatment may range from 1 to 30 days, with administration performed daily or at intervals of 2 to 5 days. If necessary, the same treatment may be repeated after an appropriate period of time. For non-human animals, the dosage may be the same per kg as for humans, or an amount converted from the human dosage based on, for example, the volume ratio (e.g., average value) of organs (such as the heart) between the target animal and humans may be administered.

The administration route of the pharmaceutical composition may be through any general route, so long as the drug can reach the target tissue. For example, the administration may be oral or parenteral administration, and may include, but is not limited to: ocular local administration (e.g., periocular (e.g., sub-Tenon's), subconjunctival, intraocular, intravitreal, intracameral, subretinal, suprachoroidal, and retrobulbar administration), intraperitoneal administration, intravenous administration, intramuscular administration, subcutaneous administration, intradermal administration, oral administration, topical administration, nasal administration, pulmonary administration, and rectal administration. Additionally, the pharmaceutical composition of the present disclosure may be administered by any device capable of transporting the active ingredient to a target cell. Preferably, the administration route of the pharmaceutical composition of the present disclosure is determined depending on the type of the disease to which it is applied.

The pharmaceutical composition of the present disclosure may be formulated according to conventional methods and used in oral dosage forms such as powders, granules, tablets, capsules, suspensions, emulsions, syrups, and aerosols, or in parenteral dosage forms such as suspensions, emulsions, lyophilized preparations, external preparations, suppositories, sterile injectable solutions, and implantable preparations. The pharmaceutical composition may further include pharmaceutically acceptable excipients usable for formulation in addition to the active ingredient.

The excipient may include one or more selected from the group consisting of carriers, vehicles, diluents, and solvents, for example, monohydric alcohols, such as ethanol and isopropanol, polyhydric alcohols, such as glycerol, and edible oils, such as soybean oil, coconut oil, olive oil, safflower oil, cottonseed oil, and oily esters, such as ethyl oleate and isopropyl myristate; binders, adjuvants, solubilizers, thickeners, stabilizers, disintegrants, glidants, lubricants, buffers, emulsifiers, wetting agents, suspending agents, sweeteners, colorants, flavoring agents, coating agents, preservatives, antioxidants, processing agents, drug delivery modifiers, and enhancers, such as calcium phosphate, magnesium stearate, talc, monosaccharides, disaccharides, starch, gelatin, cellulose, methyl cellulose, sodium carboxymethyl cellulose, dextrose, hydroxypropyl-betacyclodextrin, polyvinylpyrrolidone, low-melting point waxes, and ion exchange resins, but is not limited thereto.

The carrier includes those commonly used in formulation, including, but not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, and mineral oil. In addition to the above ingredients, the pharmaceutical composition of the present disclosure may further include a lubricant, a wetting agent, a sweetener, a flavoring agent, an emulsifier, a suspending agent, a preservative, and the like. Suitable pharmaceutically acceptable carriers and formulations are described in detail in Remington's Pharmaceutical Sciences (19th ed., 1995).

Formulating the pharmaceutical composition of the present disclosure into a parenteral dosage form may mean that the composition is administered by methods such as subcutaneous injection, intravenous injection, intramuscular injection, or intrathoracic injection. To formulate such a parenteral dosage form, the active ingredient may be mixed with a stabilizer or a buffer in water to prepare a solution or a suspension, and such a solution or suspension may be prepared in a unit dosage form such as an ampoule or a vial.

Formulating the pharmaceutical composition of the present disclosure into an oral dosage form may mean that the composition is, for example, a tablet, a pill, a hard or soft capsule, a liquid, a suspension, an emulsion, a syrup, a granule, an elixir, or the like. According to the conventional constitution of each dosage form, these oral dosage forms may include, in addition to the active ingredient, a pharmaceutically acceptable carrier such as a diluent (e.g., lactose, dextrose, sucrose, mannitol, sorbitol, cellulose, and/or glycine) or a lubricant (e.g., silica, talc, stearic acid and its magnesium or calcium salt, and/or polyethylene glycol).

When the oral dosage form is a tablet, it may include a binder such as magnesium aluminum silicate, starch paste, gelatin, tragacanth, methylcellulose, sodium carboxymethyl cellulose, and/or polyvinylpyrrolidine; and optionally, a disintegrant such as starch, agar, alginic acid or a sodium salt thereof, and/or an effervescent mixture and/or an absorbent, a colorant, a flavoring agent, or a sweetener.

Additionally, the pharmaceutical composition may be sterile, or may further include an auxiliary agent such as a preservative, a stabilizer, a wetting agent or an emulsification accelerator, a salt for regulating osmotic pressure, and/or a buffer; it may also further include other therapeutically useful substances, and may be formulated according to conventional methods of mixing, granulating, or coating.

The content of the antibody in the pharmaceutical composition of the present disclosure may be appropriately adjusted depending on the purpose of use of the pharmaceutical composition, the type of formulation, and the like, and may be, for example, 0.001 to 99 wt%, 0.001 to 90 wt%, 0.001 to 50 wt%, 0.01 to 50 wt%, 0.1 to 50 wt%, or 1 to 50 wt%, based on the total weight of the pharmaceutical composition.

Another aspect provides a health functional food composition for the prevention or improvement of cancer, including the anti-CD24 antibody or the antigen-binding fragment thereof.

The health functional food composition may be provided for the prevention, improvement, or treatment of cancer.

As used herein, the term 'health functional food' refers to food manufactured or processed for the purpose of health supplementation by using specific ingredients as raw materials or by extracting, concentrating, purifying, or mixing specific ingredients contained in food raw materials, wherein the food is designed and processed such that bioregulatory functions, including biological defense, regulation of biological rhythms, and prevention of and recovery from diseases, can be sufficiently exerted on the body by said ingredients.

There is no particular limitation on the type of the food. Examples of the food include formulations selected from the group consisting of powders, granules, tablets, capsules, pills, gels, jellies, suspensions, emulsions, syrups, tea bags, infusion teas, gums, candies, and health beverages, and include all health foods in a conventional sense.

The health functional food may include food-acceptable food additives and suitable carriers commonly used in the manufacture of health functional foods.

The effective dosage of the antibody or antigen-binding fragment thereof contained in the health functional food composition may be used based on the effective dosage of the pharmaceutical composition; however, in the case of longterm intake for the purpose of health and hygiene or health regulation, the dosage may be below the aforementioned range. Because the active ingredient poses no safety concerns, the dosage may also be used in an amount exceeding the aforementioned range.

While the present disclosure may be subject to various modifications and may have various embodiments, specific embodiments are illustrated by way of example in the drawings and will herein be described in detail. It should be understood, however, that there is no intent to limit the disclosure to the particular forms disclosed, but on the contrary, the disclosure is to cover all modifications, equivalents, and alternatives falling within the spirit and scope of the disclosure. In the description, descriptions of related well-known art may be omitted where they would obscure the disclosure with unnecessary detail.

### Advantageous Effects of Invention

According to an aspect, the CD24-specific antibody or antigen-binding fragment thereof increases the phagocytic activity of intratumoral macrophages, thereby enabling its utilization as a broad-spectrum anticancer agent for effectively preventing or treating various CD24-related cancers regardless of the specific cancer type; and has high binding affinity for both human and mouse CD24, enabling its application in both preclinical and clinical settings.

### Brief Description of Drawings

FIG. 1 is a schematic diagram illustrating a selection process and development strategy for an anti-CD24 antibody.
FIGS. 2 and 3 are graphs showing monoclonal phage ELISA results for antibody selection from a round 3 polyclonal pool.
FIG. 4 is a graph analyzing binding affinity of an anti-CD24 antibody for a breast cancer cell line according to an embodiment.
FIG. 5 is a graph analyzing binding affinity of an anti-CD24 antibody for human and mouse breast cancer, lung cancer, liver cancer, and melanoma cell lines according to an embodiment.
FIG. 6 is a schematic diagram illustrating an anti-CD24 antibody and phagocytic activity thereof according to an embodiment.
FIG. 7 is a graph showing flow cytometry analysis of macrophage phagocytic activity in a lung cancer cell line following administration of an anti-CD24 antibody according to an embodiment.
FIG. 8 is a graph showing flow cytometry analysis of macrophage phagocytic activity in a breast cancer cell line following administration of an anti-CD24 antibody according to an embodiment.

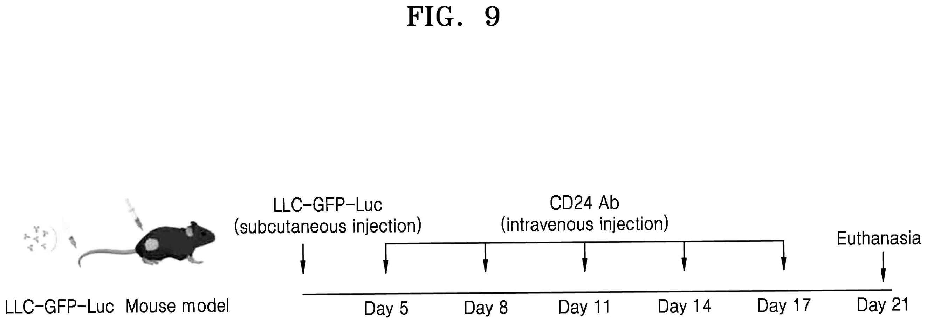

FIG. 9 is a schematic diagram illustrating construction of a tumor mouse model and an antibody treatment experiment.
FIG. 10 is a graph showing tumor volume and weight measured after treatment with an anti-CD24 antibody according to an embodiment in a tumor mouse model.
FIG. 11 is a graph showing bioluminescence signals measured after treatment with an anti-CD24 antibody according to an embodiment in a tumor mouse model.
FIG. 12 shows results of flow cytometry analysis of cancer cells within macrophages after treatment with an anti-CD24 antibody according to an embodiment in a tumor mouse model.
FIG. 13 is a graph showing changes in ratio of M1 and M2 macrophages after treatment with an anti-CD24 antibody according to an embodiment in a tumor mouse model.
FIG. 14 is a graph showing changes in expression of TNFα marker in macrophages after treatment with an anti-CD24 antibody according to an embodiment in a tumor mouse model.
FIG. 15 is a graph showing changes in expression of Granzyme B and IFNγ markers in cytotoxic T cells after treatment with an anti-CD24 antibody according to an embodiment in a tumor mouse model.

### Mode for the Invention

The present disclosure will be described in more detail with reference to the following examples. However, these examples are provided to illustratively describe one or more embodiments and are not intended to limit the scope of the present disclosure.

### Example 1. Biopanning

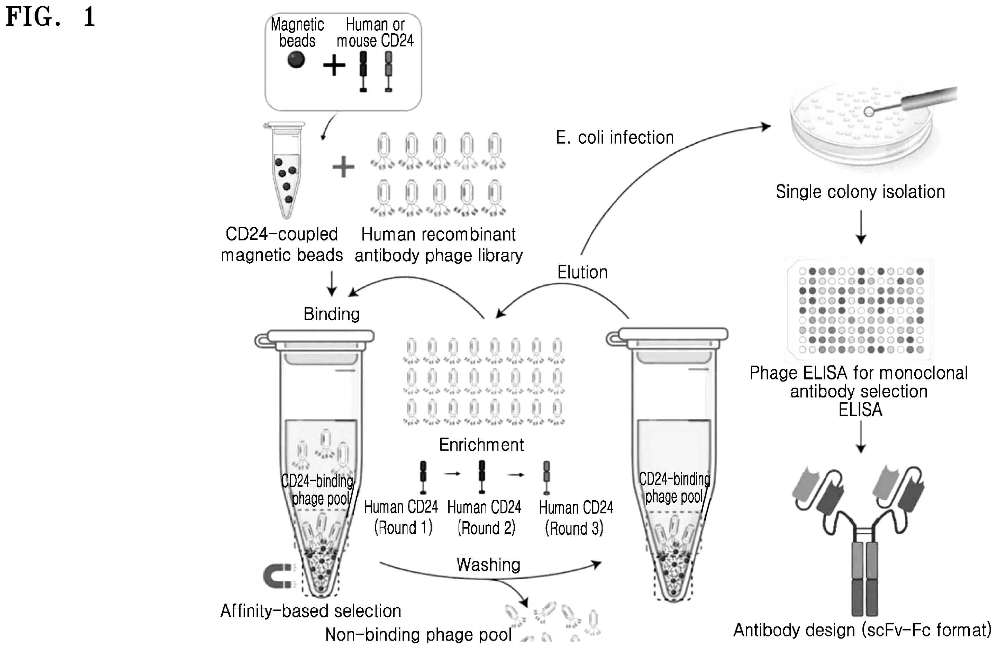

Candidate anti-CD24 antibodies were selected from a human antibody phage library via biopanning. FIG. 1 is a schematic diagram showing the selection process and development strategy for the anti-CD24 antibody.

Specifically, solution-phase panning was performed using recombinant human CD24 protein (#CD4-H52H3, Acro Biosystems), recombinant mouse CD24 protein (#CD4-M52H7, Acro Biosystems), and Dynabeads M-270 epoxy beads (#14301, Invitrogen; Thermo Fisher Scientific). The beads were incubated at 37 °C overnight for conjugation with the human or mouse CD24 protein. Each panning round was performed by alternating the conjugated beads. Then, the protein-conjugated beads were exposed to a human combinatorial antibody phage display library at 25 °C for 2 hours. After incubation, the beads were washed with PBST (0.05 % Tween 20 in phosphate-buffered saline (PBS, Cytiva)), and the bound phages were eluted at pH 2.2 using 0.2 M glycine-HCl containing 0.1 % bovine serum albumin (BSA). The collected phages were then used to infect E. coli XL1-Blue competent cells in the presence of 100 mg/mL carbenicillin and were amplified by incubation at 37 °C for 2 hours. The culture medium was then diluted in Super Broth (SB) medium, and M13KO7 helper phages (#N0315S; New England Biolabs) were added, followed by further incubation at 37 °C for 2 hours. Thereafter, 70 µg/mL of kanamycin sulfate was added, and the culture was maintained at 37 °C overnight. On the following day, the phage particles were concentrated via polyethylene glycol/NaCl precipitation, purified, and resuspended in tris-buffered saline containing 1 % BSA.

### Example 2. Enzyme-Linked Immunosorbent Assay (ELISA)

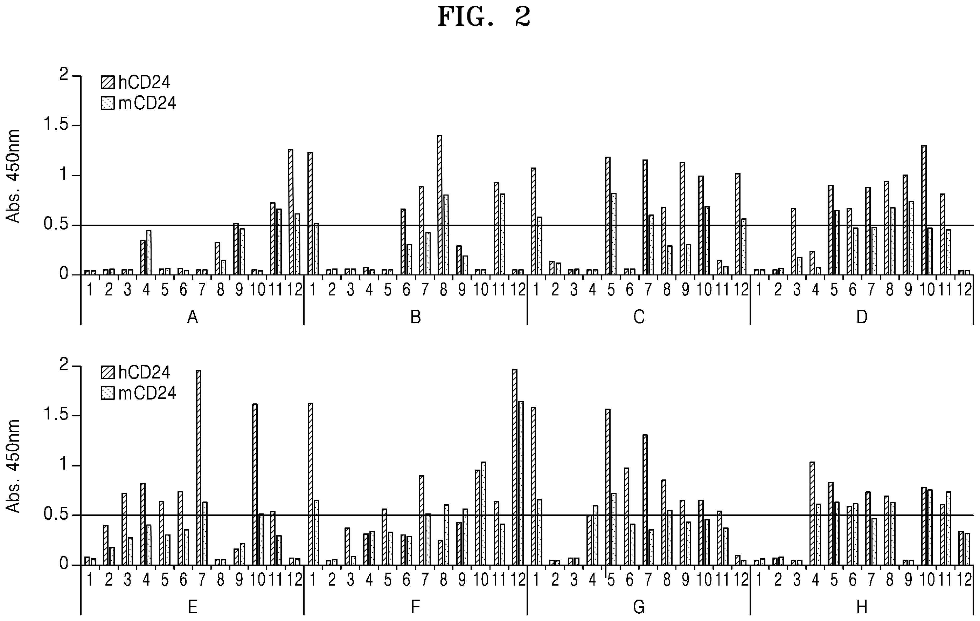

A 96-well half-area plate (#3690, Corning) was prepared by coating with recombinant human CD24 protein, recombinant mouse CD24 protein, and BSA. The plate was incubated at 4 °C overnight using a carbonate coating buffer. The plates were washed once with PBS and blocked with 5 % skim milk in PBS at 37 °C for 1 hour. After blocking, the phage supernatant was added to the plate and incubated for 2 hours at 37 °C. For the phage supernatant, the phage pool obtained from the biopanning in Example 1 was used to infect E. coli, and individual single colonies grown on carbenicillin-resistant plates were individually inoculated and cultured in a 96-deep-well plate (#90060, Bioneer) containing 1 mL of SB medium per well for 4 hours at 37 °C. Thereafter, M13KO7 helper phages were added to each well, and the resulting phage supernatant produced in each well during the overnight culture was used. After addition of the phage supernatant, each well was washed five times with PBS. After washing, HRP-tagged anti-M13 bacteriophage antibodies (#11973-MM05T-H, Sino Biological) were applied, followed by further incubation at 37 °C for 1 hour. Each well was washed five times with PBS again, and tetramethylbenzidine (TMB) was added for 10 minutes for color development, and the reaction was stopped with 2N sulfuric acid. The absorbance of each well was measured at 450 nm using a microplate reader, and phages exhibiting an absorbance value of at least 0.5 for both the recombinant human CD24 protein and the recombinant mouse CD24 protein were selected (FIG. 2).

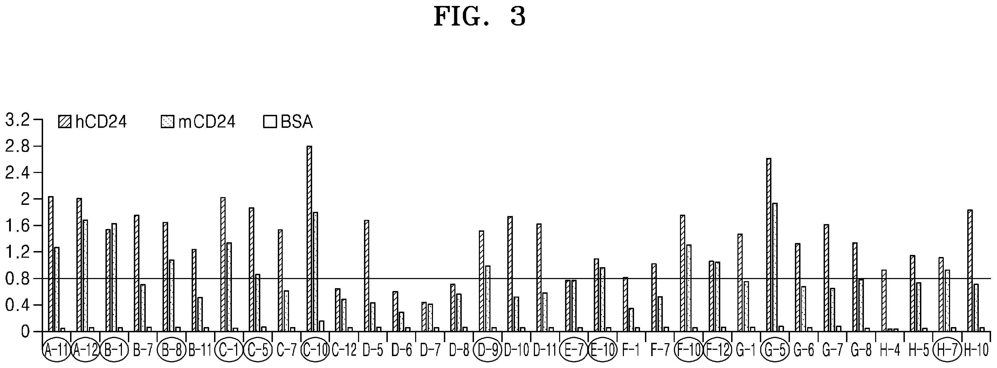

The selected phages were subjected to re-validation of their binding affinity, and phages exhibiting an absorbance value of at least 0.8 for both the recombinant human CD24 protein and the recombinant mouse CD24 protein were selected (FIG. 3). DNA sequencing of a single well from the selected phages revealed a consensus H-CDR3 sequence, as shown in Table 1. Table 2 presents the CDR sequences of the two final candidates.

**[Table 1]**

| **Clone** | **H-CDR3** |
|---|---|
| A-12 | CARDSSSWYGGYYYYYGMDVW |
| B-1 | CAREYGDYFDYW |
| B-8 | CARDSSSWYGGYYYYYGMDVW |
| C-1 | CAREYGDYFDYW |
| C-5 | CAREYGDYFDYW |
| C-10 | CAREYGDYFDYW |
| F-10 | CAREYGDYFDYW |
| G-5 | CARDSSSWYGGYYYYYGMDVW |

**[Table 2]**

| **Candidate** | | **CDR1** | **CDR2** | **CDR3** |
|---|---|---|---|---|
| 1 | Heavy chain | GFTFSSYG (SEQ ID NO: 1) | MSGDGLAT (SEQ ID NO: 3) | CARDSSSWYGGYYYYYGM DVW (SEQ ID NO: 5) |
| | Light chain | QGISSW (SEQ ID NO: 7) | AAS | CQQSYSTPLTF (SEQ ID NO: 9) |
| 2 | Heavy chain | GFTVSSNY (SEQ ID NO: 2) | IYSGGST (SEQ ID NO: 4) | CAREYGDYFDYW (SEQ ID NO: 6) |
| | Light chain | SSDVGGYDH (SEQ ID NO: 8) | EVT | CSSYAGSNSVLF (SEQ ID NO: 10) |

### Example 3. Antibody Purification and Production

The antibodies having the CDR sequences identified in Example 2 were produced and purified.

Specifically, the scFv antibody genes (SEQ ID NO: 20 or 21) were inserted into a pFUSE-Fc expression vector (#pfuse-hg1fc2; InvivoGen). Expi293F cells (#A14524; Gibco; Thermo Fisher Scientific) were then transfected using an ExpiFectamine 293 Transfection Kit (#A14527; Gibco; Thermo Fisher Scientific). After 3 days, the cell culture supernatant was filtered through a 0.2 µm filter. The filtered supernatant was then purified using a HiTrap Protein G HP column (#17-0404-01, Cytiva) on an AKTA^{™} start protein purification system (Cytiva).

### Example 4. Validation of CD24 Binding Affinity

The binding affinity of an anti-CD24 antibody according to an embodiment was evaluated against CD24 expressed in various cancer cell lines.

First, lung cancer cell lines (A549 and LLC), breast cancer cell lines (4T1 and MCF7), a liver cancer cell line (Huh7), and a melanoma cell line (B16F10) were maintained in a humidified incubator at 37 °C with 5 % CO₂. The LLC, LLC-luc, Huh7, 4T1, and B16F10 cells were cultured in Dulbecco's Modified Eagle's Medium (DMEM, Cytiva) supplemented with 10 % fetal bovine serum (FBS, Cytiva) and 1 % antibiotic-antimycotic (AA, GenDEPOT). The A549 and MCF7 cells were cultured in Roswell Park Memorial Institute medium (RPMI1640, Cytiva) containing 10 % FBS and 1 % AA.

All cell lines utilized in the study were washed with PBS and then fixed with 4 % paraformaldehyde (PFA) at 4 °C for 10 minutes. After fixation, the cells were washed again and blocked with 5 % goat serum and 1 % BSA in PBS-T. Cell staining was performed with the designated primary antibody at 4 °C for 1 hour, followed by further incubation with an appropriate secondary antibody at the same temperature for 1 hour. The previously purified anti-CD24 antibody was used as the primary antibody. FITC-conjugated goat anti-human IgG antibodies (#ab97224, Abcam) were used as secondary antibodies. Sample analysis was performed using a CytoFLEX flow cytometer (Beckman Coulter), and the data were processed using FlowJo software.

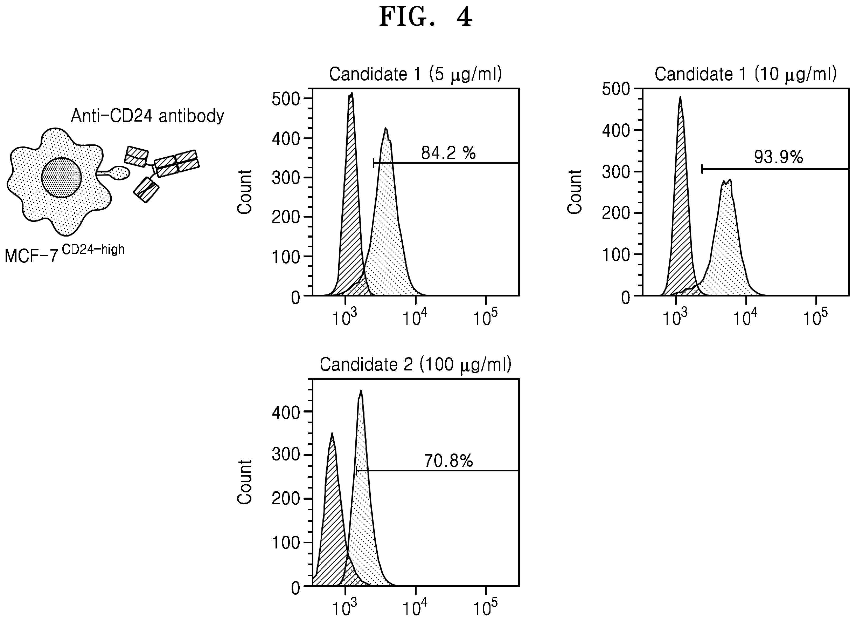

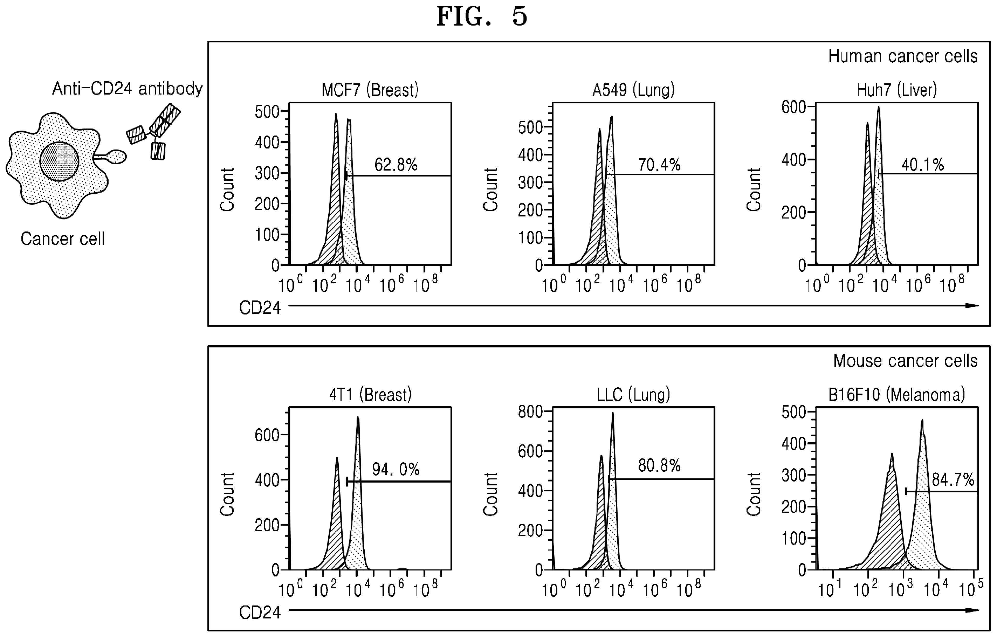

As shown in FIG. 4 and FIG. 5, antibodies #1 and #2 of the present disclosure were found to exhibit high binding affinity to CD24 in the MCF-7 breast cancer cell line. Additionally, antibody #1 was confirmed to exhibit high binding affinity to CD24 across human and mouse cancer cell lines, including breast cancer (MCF7 and 4T1), lung cancer (A549 and LLC), liver cancer (Huh7), and melanoma (B16F10).

### Example 5. Phagocytosis Analysis

The involvement of an anti-CD24 antibody according to an embodiment in macrophage phagocytosis was evaluated through the following experiment.

Specifically, THP-1 cells (Korean Cell Line Bank) (5 × 10⁵ cells/well) were seeded in a 6-well plate containing 2 mL of RPMI medium (RPMI1640, Cytiva) containing 10 % FBS and 1 % AA. To optimize differentiation conditions, the THP-1 cells were treated with 50 nM phorbol 12-myristate 13-acetate (PMA, Sigma) for 48 hours. The differentiated adherent cells were washed twice with PBS and rested for an additional 24 hours in culture medium to reach the resting state (M0) of macrophages. The M0 macrophages were stimulated with IL-4/IL-13 at a concentration of 20 ng/mL for 72 hours to induce differentiation into the M2 phenotype, which was subsequently utilized for phagocytosis analysis.

All flow-based *in vitro* phagocytosis assays were performed by co-culturing cancer cells and macrophages at a ratio of 1x10⁵ target cells to 5x10⁴ macrophages in ultra-low attachment 96-well U-bottom plates (Costar) in serumfree RPMI for 4 hours in a humidified 5 % CO₂ incubator at 37 °C. The cancer cells were labeled with 0.5 µM carboxyfluorescein succinimidyl ester (CFSE, Invitrogen) according to the manufacturer's instructions, incubated at 37 °C for 20 minutes with protection from light, and washed twice with culture medium prior to co-culture. The cancer cells were pre-treated with an anti-CD24 antibody (#1) according to an embodiment, and the macrophages were harvested from the plates using TrypLE Express (Gibco). After co-culture, phagocytosis was analyzed by flow cytometry in the same manner as in Example 4. The macrophages were identified with an anti-CD11b-APC antibody (#17-0112-82, 1:400, Invitrogen), and the phagocytosis percentage was determined based on CD11b+/CSFE+ macrophages using a CytoFLEX flow cytometer (Beckman Coulter), and the data were analyzed using FlowJo software.

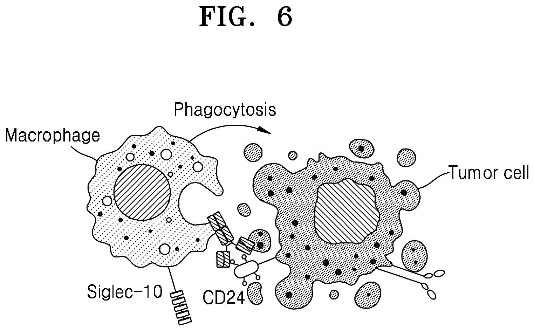

FIG. 6 is a schematic diagram illustrating an anti-CD24 antibody according to an embodiment and its phagocytic activity.

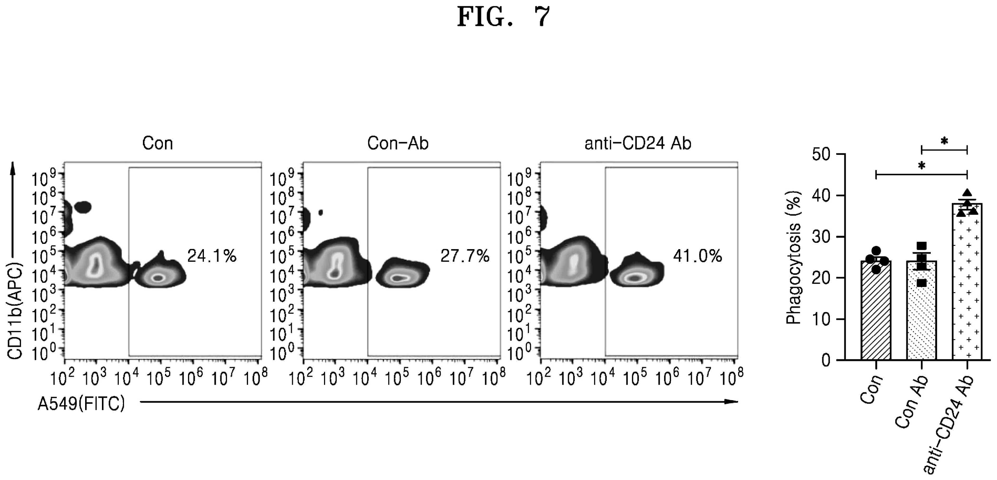

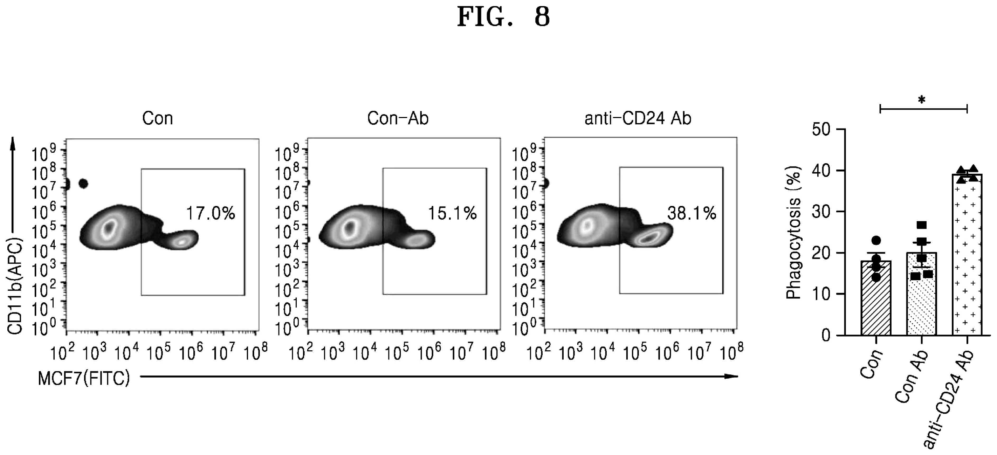

As shown in FIG. 7 and FIG. 8, it was confirmed that the group treated with antibody #1 exhibited increased macrophage phagocytic activity in both lung and breast cancer cell lines.

### Example 6. In Vivo Anti-tumor Efficacy Validation

The anti-tumor efficacy of an anti-CD24 antibody according to an embodiment was evaluated in a mouse model.

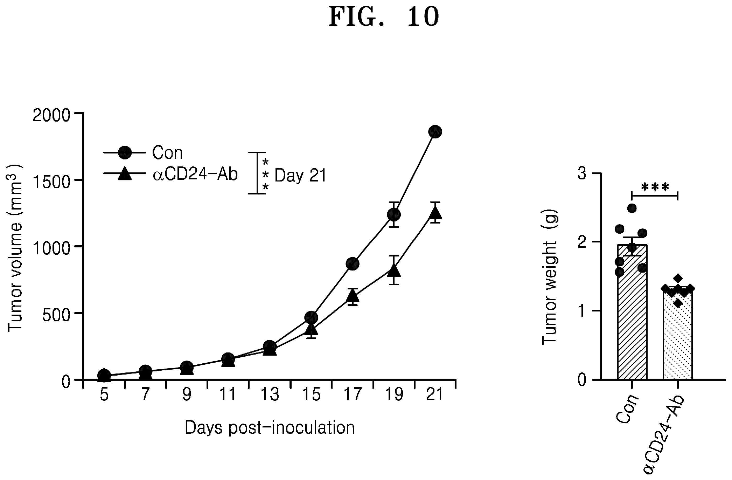

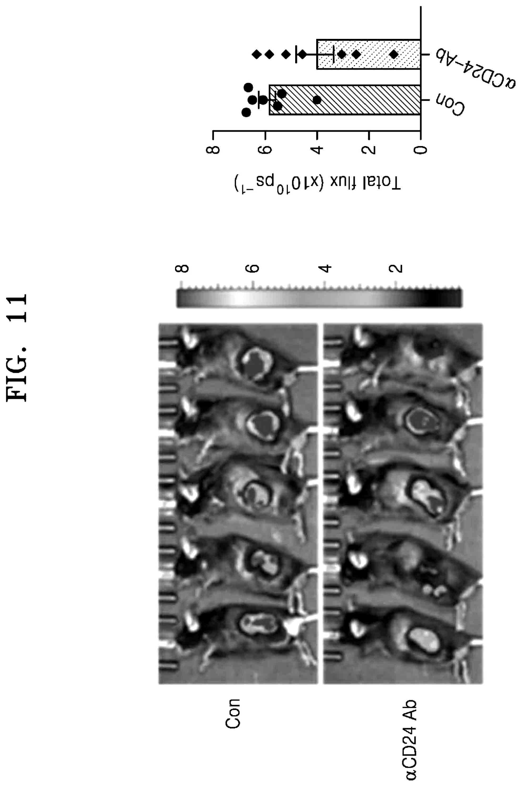

First, 1 × 10⁶ LLC-GFP-luc cells were subcutaneously inoculated into the left flank of C57BL/6 mice. On day 5, mice with palpable tumors of approximately 30 mm³ were randomly divided into two groups, and PBS and an αCD24 antibody (#1) were administered via intravenous injection. The αCD24 antibody (200 µg/mouse) was injected at three-day intervals. Tumor growth was measured every 2 days with digital calipers, and tumor size was calculated using the ellipsoid formula V = ½ length × width². According to humane endpoint criteria, euthanasia was performed when signs of distress appeared in the mice or the tumor volume exceeded 2,000 mm³. Immediately before euthanasia, *in vivo* bioluminescence levels were measured using an In Vivo Imaging System (IVIS) following administration of D-luciferin (#122799, Perkinelmer). The weight of the excised tumor tissue was measured, with the results shown in FIG. 10 and FIG. 11.

As shown in FIG. 10, it was confirmed that the tumor weight and volume in the group treated with the #1 anti-CD24 antibody were reduced compared to those of the control group.

As shown in FIG. 11, it was confirmed that the bioluminescence signal in the group treated with the #1 anti-CD24 antibody was suppressed compared to that of the control group.

These results demonstrate that tumor growth is inhibited by the anti-CD24 antibody according to an embodiment.

### Example 7. In Vivo Flow Cytometry Analysis

The increase in macrophage activity following the administration of an anti-CD24 antibody according to an embodiment was evaluated via flow cytometry analysis.

First, cells were isolated from tumor tissue for flow cytometry analysis. Mice were prepared in the same manner as described in Example 6, and tumor tissues were excised from the LLC tumor-bearing mice. The weight of each tissue was measured. A 40 µm cell strainer was placed on a 50 mL conical tube, and the tissue was mashed through the strainer using the plunger of a 3 mL syringe. The sample was washed with PBS and resuspended in 5 mL of RBC lysis buffer (Sigma-Aldrich), followed by incubation for 5 minutes. Additionally, 25 mL of PBS was added, and after washing, the cells were harvested by centrifugation at 550×g. The cells were fixed in 4 % PFA and subsequently used for flow cytometry analysis.

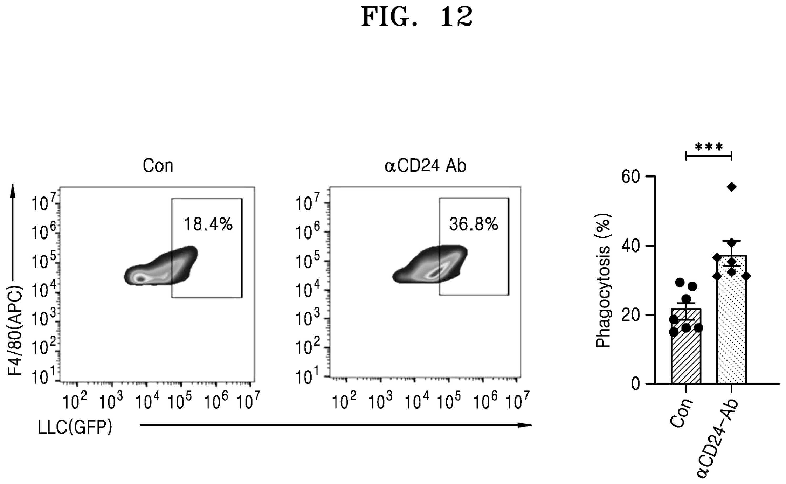

For *in vivo* phagocytosis analysis, non-specific binding to FcR was reduced by incubation with 2 µl of TruStain FcX Fc receptor blocking antibody (BioLegend) on ice for 10 minutes. Thereafter, intratumoral macrophages were analyzed by staining with CD45-PE-Cy7 (#60943S, Cell signaling) and anti-F4/80-APC antibodies (#86007S, Cell signaling). Samples were analyzed on a CytoFLEX flow cytometer (Beckman Coulter), and the data were processed with FlowJo software (FIG. 12).

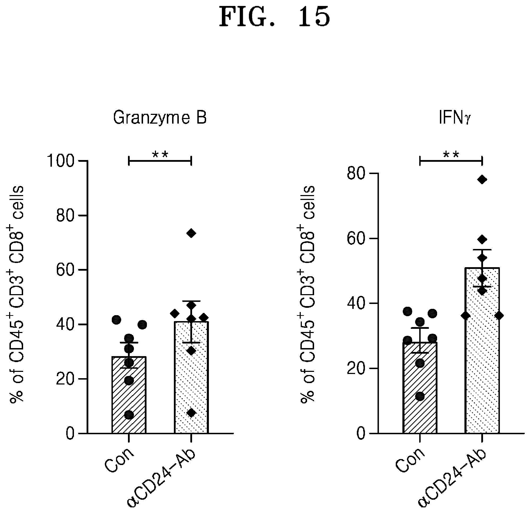

For *in vivo* immunoanalysis, non-specific binding to FcR was reduced by incubation with 2 µl of TruStain FcX Fc receptor blocking antibody (BioLegend) on ice for 10 minutes. Thereafter, to analyze intratumoral macrophages, the frequency and activity of M1-TAMs (CD45+CD11b+F4/80+CD80+) and M2-TAMs (CD45+CD11b+F4/80+CD206+) were evaluated using anti-CD45-PE-Cy7 (#60943S, Cell signaling), anti-CD11b-PE (#24956S, Cell signaling), anti-F4/80-APC (#86007S, Cell signaling), anti-CD80-eFluor^{™} 450 (#48-0801-82, Invitrogen), anti-CD206 eFluor^{™} 450 (#48-2061-82, Invitrogen), and anti-TNFα-eFluor^{™} 450 (#48-7321-82, Invitrogen). To analyze intratumoral T cells, CD8+ T cell activity was evaluated using anti-CD45-PE-Cy7 (#60943S, Cell signaling), anti-CD3-APC (#24265S, Cell signaling), anti-CD8α-PE (#56984S, Cell signaling), anti-IFN-γ (#ab9657, Abcam), anti-Granzyme B (#ab4059, Abcam), and Rabbit IgG-Pacific Blue (#P-10994, Invitrogen). Samples were analyzed on a CytoFLEX flow cytometer (Beckman Coulter), and the data were processed with FlowJo software (FIG. 15).

As shown in FIG. 12, an analysis of cancer cells (GFP) phagocytosed by macrophages (F4/80) confirmed that the intratumoral phagocytic capacity for cancer cells was significantly improved compared to the control group.

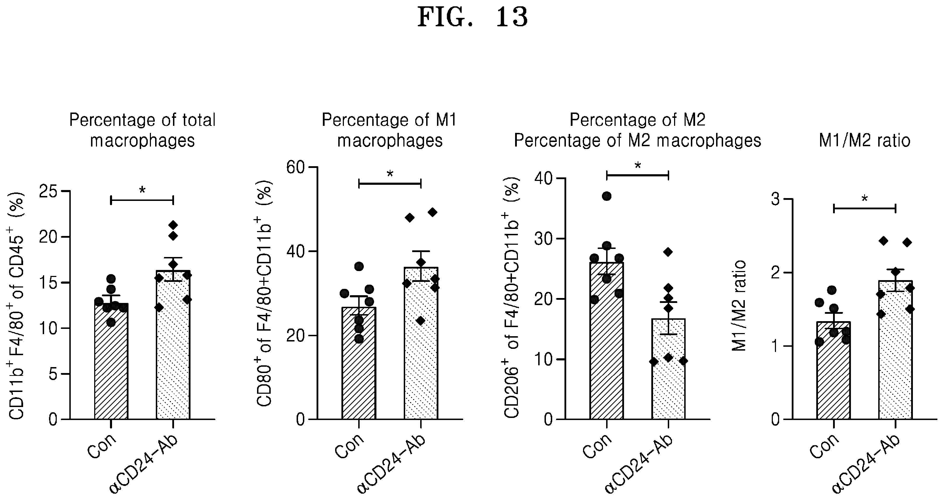

As shown in FIG. 13, it was confirmed that macrophage infiltration into the tumor tissue was increased, with an increased ratio of anti-tumor macrophages (M1-TAMs) relative to pro-tumor macrophages (M2-TAMs).

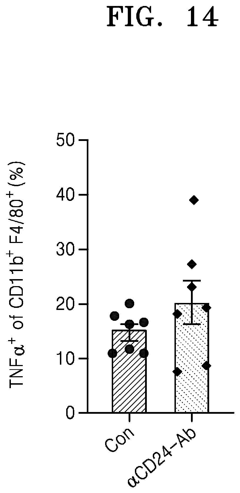

As shown in FIG. 14, an increase in the expression of TNFα, a representative activation marker of anti-tumor M1 macrophages, was confirmed.

As shown in FIG. 15, increased expression of Granzyme B and IFNγ, which are representative activation markers of cytotoxic T cells (CD8+ T cells) resulting from intratumoral macrophage activation, was confirmed.

The above results indicate that intratumoral macrophages are activated by the anti-CD24 antibody according to an embodiment.

In summary, the anti-CD24 antibody of the present disclosure not only targets cancer cells but also blocks the "don't eat me" signal to enhance the phagocytic capacity of macrophages against cancer cells. Accordingly, the antibody can be utilized as a broad-spectrum anticancer agent for effectively preventing or treating various CD24-related cancers regardless of the specific cancer type. An increase in the activity of T cells and NK cells expressing Siglec-10, which interacts with CD24, is also anticipated, thereby providing a novel cancer immunotherapy strategy. In addition, the high binding affinity of the antibody for both human and mouse CD24 enables its application in both preclinical and clinical settings.

The foregoing description is illustrative of the present disclosure, and it will be apparent to those skilled in the art that various changes and modifications may be made without departing from the spirit and scope of the disclosure. Accordingly, the embodiments disclosed herein are intended to be illustrative rather than limiting, and the scope of the disclosure is defined by the appended claims rather than by the foregoing description.

## Claims

1. An antibody or antigen-binding fragment thereof that specifically binds to CD24, comprising:
a heavy chain variable region comprising a heavy chain complementarity determining region 1 (H-CDR1) comprising an amino acid sequence of SEQ ID NO: 1 or 2, a heavy chain complementarity determining region 2 (H-CDR2) comprising an amino acid sequence of SEQ ID NO: 3 or 4, and a heavy chain complementarity determining region 3 (H-CDR3) comprising an amino acid sequence of SEQ ID NO: 5 or 6; and
a light chain variable region comprising a light chain complementarity determining region 1 (L-CDR1) comprising an amino acid sequence of SEQ ID NO: 7 or 8, a light chain complementarity determining region 2 (L-CDR2) comprising an amino acid sequence of AAS or EVT, and a light chain complementarity determining region 3 (L-CDR3) comprising an amino acid sequence of SEQ ID NO: 9 or 10.

2. The antibody or antigen-binding fragment thereof according to claim 1, wherein the heavy chain variable region comprises a combination of (i) or (ii):
(i) an H-CDR1 comprising an amino acid sequence of SEQ ID NO: 1; an H-CDR2 comprising an amino acid sequence of SEQ ID NO: 3; and an H-CDR3 comprising an amino acid sequence of SEQ ID NO: 5;
(ii) an H-CDR1 comprising an amino acid sequence of SEQ ID NO: 2; an H-CDR2 comprising an amino acid sequence of SEQ ID NO: 4; and an H-CDR3 comprising an amino acid sequence of SEQ ID NO: 6.

3. The antibody or antigen-binding fragment thereof according to claim 1, wherein the light chain variable region comprises a combination of (i) or (ii):
(i) an L-CDR1 comprising an amino acid sequence of SEQ ID NO: 7; an L-CDR2 comprising an amino acid sequence of AAS; and an L-CDR3 comprising an amino acid sequence of SEQ ID NO: 9;
(ii) an L-CDR1 comprising an amino acid sequence of SEQ ID NO: 8; an L-CDR2 comprising an amino acid sequence of EVT; and an L-CDR3 comprising an amino acid sequence of SEQ ID NO: 10.

4. The antibody or antigen-binding fragment thereof according to claim 1, comprising:
a heavy chain variable region comprising an amino acid sequence of SEQ ID NO: 11 or 12; and
a light chain variable region comprising an amino acid sequence of SEQ ID NO: 13 or 14.

5. The antibody or antigen-binding fragment thereof according to claim 1, comprising one or more amino acid sequences selected from the group consisting of SEQ ID NOs: 15 to 18.

6. The antibody or antigen-binding fragment thereof according to claim 1, wherein the heavy chain variable region and the light chain variable region are linked directly or via a linker.

7. The antibody or antigen-binding fragment thereof according to claim 1, wherein the antibody or antigen-binding fragment thereof comprises an scFv or an scFv-Fc fragment.

8. The antibody or antigen-binding fragment thereof according to claim 1, wherein the antibody or antigen-binding fragment thereof increases phagocytic activity of macrophages.

9. The antibody or antigen-binding fragment thereof according to claim 1, wherein the antibody or antigen-binding fragment thereof is a mouse antibody, a chimeric antibody, a humanized antibody, or a fully human antibody.

10. A gene encoding the antibody or antigen-binding fragment thereof according to any one of claims 1 to 9.

11. A vector comprising the gene according to claim 10.

12. A recombinant cell comprising the gene according to claim 10 or a vector comprising the gene.

13. An antibody-drug conjugate (ADC) comprising:
the antibody or antigen-binding fragment thereof according to claim 1;
a drug moiety; and
a linker linking the antibody or antigen-binding fragment thereof and the drug moiety.

14. A pharmaceutical composition for preventing or treating cancer, comprising: the antibody or antigen-binding fragment thereof according to any one of claims 1 to 9; or the antibody-drug conjugate according to claim 13.

15. The pharmaceutical composition according to claim 14, wherein the cancer comprises one or more selected from the group consisting of breast cancer, lung cancer, skin cancer, kidney cancer, colorectal cancer, head and neck cancer, stomach cancer, colon cancer, prostate cancer, bladder cancer, rectal cancer, thyroid cancer, liver cancer, cervical cancer, melanoma, rectal cancer, anal cancer, urethral cancer, ovarian cancer, esophageal cancer, and pancreatic cancer.

16. A method of preventing or treating cancer, comprising administering an effective amount of the antibody or antigen-binding fragment thereof according to any one of claims 1 to 9; or the antibody-drug conjugate according to claim 13, to a subject in need thereof.

17. Use of the antibody or antigen-binding fragment thereof according to any one of claims 1 to 9; or the antibody-drug conjugate according to claim 13, in the manufacture of a pharmaceutical preparation for preventing or treating cancer.
